# FASCICULE DE BREVET EUROPEEN

(11) **EP 2 946 018 B1**
(45) Date de publication et mention de la délivrance du brevet: **17.05.2017**
(21) Numéro de dépôt: 14701334.6
(22) Date de dépôt: 20.01.2014
(51) Int. Cl.: C12Q 1/06, C12Q 1/24, C12M 1/26, C12M 1/30, C12M 1/22

(54) **MOYEN, PROCEDE ET PRODUIT-PROGRAMME D'ORDINATEUR POUR DETERMINER LE TAUX DE CONCENTRATION DE MICRO-ORGANISMES LORS D'UNE ANALYSE DE FLUIDE**
VORRICHTUNG, VERFAHREN UND COMPUTERPROGRAMMPRODUKT ZUR BESTIMMUNG DES KONZENTRATIONSGEHALTES VON MIKROORGANISMEN WÄHREND EINER FLÜSSIGKEITSANALYSE
MEANS, METHOD AND COMPUTER PROGRAM PRODUCT FOR DETERMINING THE CONCENTRATION LEVEL OF MICROORGANISMS DURING A FLUID ANALYSIS

(30) Priorité: 21.01.2013 FR 1350503
(43) Date de publication de la demande: 25.11.2015
(73) Titulaire: Biomérieux, 69280 Marcy l'Étoile (FR)
(72) Inventeur: DRAZEK, Laurent, F-38100 Grenoble (FR); FULCHIRON, Corine, F-01470 Serrieres de Briord (FR); PINSTON, Frédéric, F-38000 Grenoble (FR); ROSTAING, Hervé, F-38420 Le Versoud (FR)
(74) Mandataire: Richaud, Fabien
(86) Numéro de dépôt international: PCT/EP2014/050999
(87) Numéro de publication internationale: WO 2014/111559

(56) Documents cités:
- WO-A1-2005/071055
- US-A- 3 892 632

## Description

### Domaine technique

La présente invention concerne le domaine de l'analyse biologique et, plus précisément, un moyen, un procédé et un produit-programme d'ordinateur pour déterminer le taux de concentration de micro-organismes présents dans un fluide, lors de l'analyse d'un échantillon de ce fluide.

### Etat de la technique

Dans le domaine de l'analyse biologique, il existe de nombreux procédés afin de déterminer les taux de concentration de micro-organismes dans des échantillons biologiques. Ces procédés s'appliquent notamment en utilisant une boîte de Pétri et un dispositif d'ensemencement, tel qu'un peigne adapté comme décrit dans les demandes de brevet WO2005/071055 et WO2008/093439.

Ainsi, dans l'art antérieur, la boîte de Pétri comprend un milieu de culture tel qu'un milieu gélosé. Un échantillon de fluide comprenant des micro-organismes est introduit sur le milieu de culture. Le fluide est appliqué selon une bande qui s'étend du centre de la boîte de Pétri vers le bord de la boîte de Pétri. La longueur de cette bande de fluide s'étend donc dans la direction du rayon de la boîte de Pétri. Ensuite, dans le cas d'un ensemencement manuel ou automatique, un peigne, tel que décrit dans les demandes de brevet WO2005/071055 et WO2008/093439, déplace les micro-organismes, amassés au sein de la bande de fluide, sur toute la circonférence de la boîte de Pétri. Le peigne, situé au-dessus de la boîte de Pétri, peut se déplacer selon un axe perpendiculaire au plan de la boîte de Pétri, ladite boîte de Pétri pouvant décrire une rotation sur elle-même. De manière générale, la largeur du peigne correspond environ au rayon de la boîte de Pétri. Le peigne est positionné de telle sorte qu'une première dent du peigne est située au centre de la boîte de Pétri et une dernière dent du peigne est située à proximité du bord de la boîte de Pétri. Le peigne permet alors de répartir le contenu de la bande de fluide selon un angle allant de 0° à environ 330° au sein du milieu de culture.

Dans le cas d'un ensemencement manuel ou automatique, plusieurs procédés de détermination de concentration des micro-organismes sont applicables. Un de ces procédés utilise un découpage de la boîte de Pétri en huit secteurs c'est-à-dire en octants, sur un milieu ChromID™ CPS^{®} (Réf. 43541) pour l'identification et le dénombrement des germes urinaires. Ainsi, lorsque l'utilisateur souhaite évaluer le taux de concentration bactérienne du fluide réparti sur le milieu de culture, l'utilisateur estime le taux de recouvrement du milieu de culture dans chaque octant, puis utilise un abaque préalablement établi et fourni à l'utilisateur. Cette méthode est fastidieuse car l'utilisateur doit recenser toutes les colonies présentes dans tous les octants. Ce recensement peut donc engendrer des erreurs de dénombrement et par conséquent des erreurs sur le taux de concentration bactérienne à déterminer.

Lors du déplacement du peigne, la répartition du fluide sur le milieu de culture n'est pas homogène au sein d'un secteur donné, selon la dent du peigne considérée. En effet, pour un angle de rotation associé au déplacement du peigne, les dents du peigne parcourent une distance qui varie selon la localisation des dents sur le peigne. Si l'on considère des dents situées à proximité du centre de la boîte de Pétri, la distance azimutale parcourue par ces dents est inférieure à la distance azimutale parcourue par des dents situées à proximité du bord de la boîte de Pétri, et ce pour un angle de rotation identique du peigne sur le milieu de culture.

Ainsi, en appliquant la méthode qui utilise le découpage de la boîte de Pétri en huit secteurs, la distance parcourue par le fluide pour un secteur considéré dépend de la dent du peigne associée au déplacement dudit fluide. Par conséquent, chaque secteur contient une quantité de fluide dont la concentration en bactéries est variable sur la surface d'un secteur donné.

La répartition non-homogène du fluide au sein des différents secteurs génère également des erreurs de mesure lors de la détermination de la concentration des micro-organismes présents dans le fluide à analyser. Ainsi, cette méthode ne permet pas de déterminer avec précision la concentration des micro-organismes au sein du fluide.

Le brevet US 3,892,632 divulgue un procédé consistant à :
- déposer une quantité variable - mais connue - d'une solution bactérienne à la surface d'un milieu gélosé contenu dans une boîte de Pétri en rotation, en faisant varier, de manière continue, la quantité de solution déposée en forme de spirale sur ledit milieu gélosé,
- compter les colonies dans une zone déterminée de la boîte de Pétri au moyen d'une source lumineuse dont le faisceau est interrompu par la présence de colonies, et
- diviser le nombre de colonies comptées par le volume de solution déposée dans cette zone pour calculer la concentration bactérienne de la solution dans cette zone.

Cependant, le susdit procédé s'avère imprécis quant à la détermination du nombre de micro-organismes en fonction d'une zone de repérage initiale d'ensemencement.

### Exposé de l'invention

La présente invention vise à surmonter au moins partiellement les problèmes mentionnés ci-dessus.

Un objectif de la présente invention consiste à fournir un moyen de repérage pour déterminer la concentration en micro-organismes d'un fluide à analyser au moyen d'une boîte de Pétri, ladite boîte de Pétri comprenant un couvercle et un fond, le moyen de repérage étant adapté pour être posé sur le couvercle de la boîte de Pétri ou intégré au couvercle de la boîte de Pétri ou intégré au sein d'un logiciel informatique, ladite boîte de Pétri comprenant un milieu de culture sur lequel un dispositif d'ensemencement mobile en rotation par rapport à la boîte de Pétri est adapté pour répartir le fluide, ledit dispositif d'ensemencement comprenant au moins un point de contact associé à une zone de contact avec ledit milieu de culture, ledit moyen de repérage comprenant :
- un cercle C1 correspondant à la circonférence de la boîte de Pétri, le cercle C1 comprenant un centre 0 et un rayon RC1 correspondant au rayon de la boîte de Pétri;
- une ligne d'ensemencement LR de longueur R représentant la zone d'inoculation du fluide sur le milieu de culture et correspondant à une portion du rayon RC1 de la boîte de Pétri ;
- au moins une zone de repérage Zn pour repérer la présence de micro-organismes sur le milieu de culture,
   ladite zone de repérage Zn étant délimitée par la ligne d'ensemencement LR et une ligne de limite de zone LZ délimitant une distance identique d parcourue sur la surface du milieu de culture par chaque point de contact de la zone de contact du dispositif d'ensemencement à partir de la ligne d'ensemencement LR.

De manière avantageuse, le fluide comprend une concentration en micro-organismes supérieure à 10³ micro-organismes par millilitre (ml).

De manière avantageuse, chaque zone de repérage Zn correspond à une valeur prédéterminée de la concentration en micro-organismes du fluide.

De manière avantageuse, la valeur prédéterminée est définie par une fonction exponentielle de l'angle de rotation du dispositif d'ensemencement, de la longueur R de la ligne d'ensemencement et d'une constante définissant la vitesse d'épuisement de la concentration bactérienne dans le fluide en fonction de la distance parcourue sur le milieu de culture.

De manière avantageuse, la zone de repérage Zn est délimitée par le cercle C1 et par un cercle de rayon RC2 concentrique au cercle C1 et tel que RC2 est inférieur à RC1.

De manière avantageuse, la ligne de limite de zone LZ est formée par un ensemble de points Pn, chaque point Pn étant situé sur un cercle Cn concentrique au cercle C1, ledit cercle Cn comprenant un rayon RCn tel que RCn est inférieur à RC1 et supérieur à RC2, et chaque point Pn étant situé à une distance égale de la ligne d'ensemencement LR pour une même ligne de limite de zone LZ.

De manière avantageuse, les micro-organismes comprennent des bactéries.

De manière avantageuse, le milieu de culture comprend un milieu gélosé.

De manière avantageuse, le fluide est constitué par un échantillon d'origine clinique, alimentaire, environnementale, vétérinaire, pharmaceutique ou cosmétique.

Un autre objectif de l'invention consiste à fournir un procédé de repérage pour déterminer la concentration en micro-organismes d'un fluide à analyser au moyen d'une boîte de Pétri de rayon RC1, ladite boîte de Pétri comprenant un milieu de culture, ledit procédé comprenant les étapes suivantes :
- déposer le fluide sur le milieu de culture pour former une ligne d'ensemencement LR correspondant à une portion du rayon RC1 de la boîte de Pétri ;
- répartir le fluide contenu dans la ligne d'ensemencement LR sur le milieu de culture au moyen d'un dispositif d'ensemencement mobile en rotation par rapport à la boîte de Pétri, ledit dispositif d'ensemencement comprenant au moins un point de contact avec le milieu de culture ;
- incuber la boîte de Pétri pour permettre la croissance des micro-organismes sur le milieu de culture ;
- identifier des zones de repérage Zn adjacentes pour repérer la présence des micro-organismes sur le milieu de culture, lesdites zones de repérage Zn couvrant au moins une partie de la surface de la boîte de Pétri,
   et dans lequel, une première zone de repérage Zn est délimitée par la ligne d'ensemencement LR et une ligne de limite de zone LZ délimitant une même distance d parcourue sur le milieu de culture, par chaque point de contact du dispositif d'ensemencement à partir de la ligne d'ensemencement LR.

De manière avantageuse, le fluide comprend une concentration en micro-organismes supérieure à 10³ micro-organismes par millilitre (ml).

De manière avantageuse, le procédé de repérage comprend l'étape suivante :
- identifier une zone de repérage Zn comprenant des colonies isolées de micro-organismes.

De manière avantageuse, le procédé de repérage comprend l'étape suivante :
- retrouver dans une table de correspondance une valeur prédéterminée de concentration en micro-organismes associée à la zone de repérage Zn identifiée afin de déterminer la concentration des micro-organismes dans le fluide.

Un autre objectif de l'invention consiste à fournir un produit-programme d'ordinateur comprenant des instructions logicielles pour la mise en oeuvre d'un procédé de repérage ci-dessus mentionné lorsque ledit programme est exécuté par un processeur de données.

### Brève description des dessins

L'invention, sa fonctionnalité, ses applications ainsi que ses avantages seront mieux appréhendés à la lecture de la présente description, faite en référence aux figures, dans lesquelles :
- la figure 1 montre un schéma d'un moyen de repérage, selon un mode de réalisation de l'invention, à titre d'exemple ;
- la figure 2 montre un premier schéma détaillé d'un moyen de repérage en cours d'élaboration, selon un mode de réalisation de l'invention, à titre d'exemple ;
- la figure 3 montre un deuxième schéma détaillé d'un moyen de repérage en cours d'élaboration, selon un mode de réalisation de l'invention, à titre d'exemple ;
- la figure 4 montre un schéma d'un moyen de repérage intégré sur le couvercle d'une boîte de Pétri ;
- la figure 5 montre un fond d'une boîte de Pétri comprenant un milieu gélosé, selon un mode de réalisation de l'invention, à titre d'exemple ;
- la figure 6 montre un dispositif d'ensemencement, tel qu'un peigne, localisé au-dessus d'un fond d'une boîte de Pétri, selon un mode de réalisation de l'invention, à titre d'exemple ;
- la figure 7 montre une bande de fluide disposée sur le milieu de culture du fond de la boîte de Pétri, selon un mode de réalisation de l'invention, à titre d'exemple ;
- la figure 8 montre un diagramme des étapes du procédé de repérage, selon un mode de réalisation de l'invention, à titre d'exemple ;
- les figures 9, 10 et 11 montrent des exemples d'application du moyen de repérage sur le contenu de différentes boîtes de Pétri comprenant différents types de fluides avec différentes concentrations de micro-organismes ;
- les figures 12, 13 et 14 montrent des exemples d'application du moyen de repérage sur le contenu de différentes boîtes de Pétri lors de l'analyse de différents types de fluide au cours du temps.

### Description détaillée de l'invention

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après.

La présente invention concerne l'analyse d'un échantillon. Selon la présente invention, l'échantillon peut être de diverses origines, par exemple d'origine alimentaire, environnementale, vétérinaire, clinique, pharmaceutique ou cosmétique.

Parmi les échantillons d'origine alimentaire, l'on peut citer, de façon non-exhaustive, un échantillon de produits lactés (yaourts, fromages, etc.), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non transformées ou partiellement transformées. Un échantillon alimentaire peut également être issu d'une alimentation destinée aux animaux, telle que des tourteaux, des farines animales.

Tel qu'indiqué précédemment, l'échantillon peut être d'origine environnementale et peut consister, par exemple, en un prélèvement de surface, d'eau, d'air, etc.

L'échantillon peut également consister en un échantillon d'origine clinique, pouvant correspondre à des prélèvements de fluide biologique (urine, sang total, ou dérivés tel que sérum, salive, pus, liquide céphalo-rachidien, etc.), de selles (par exemple diarrhées cholériques), de prélèvements de nez, de gorge, de peau, de plaies, d'organes, de tissus ou de cellules isolées. Cette liste n'est évidemment pas exhaustive.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon prélevé est, en général, susceptible de - ou suspecté de - contenir au moins un micro-organisme externe cible tel qu'une bactérie, une levure, un champignon.

Selon la présente invention, l'analyse est réalisée à l'aide d'une boîte de Pétri comprenant un fond et un couvercle. Le fond comprend un milieu de culture tel qu'un milieu de culture gélosé. Le but de l'analyse est de déterminer la présence de micro-organismes spécifiques et la concentration en micro-organismes au sein du fluide sur le milieu de culture, après incubation de la boîte de Pétri.

La présente invention propose de déterminer la concentration des micro-organismes présents sur le milieu de culture en repérant les colonies de micro-organismes isolées sur ledit milieu de culture.

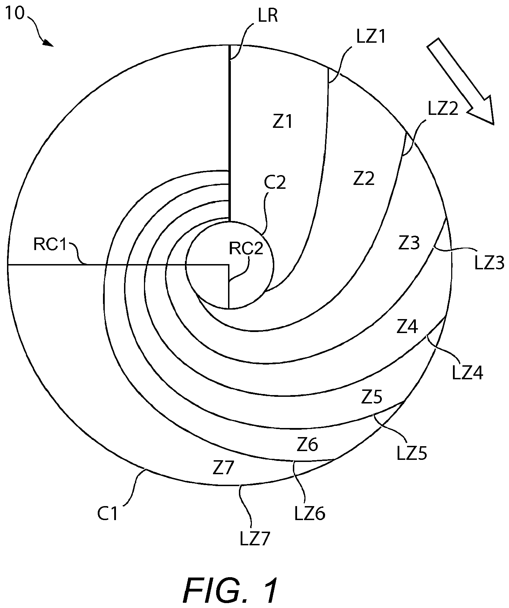

La figure 1 montre un moyen de repérage 10. Ce moyen de repérage 10 peut être réalisé dans une matière translucide afin d'être posé sur le couvercle d'une boîte de Pétri pour observer le milieu de culture gélosé comprenant les micro-organismes. Le moyen de repérage 10 peut également être intégré au couvercle d'une boîte de Pétri au moyen d'un procédé graphique par exemple. Enfin, le moyen de repérage 10 peut également être intégré au sein d'un logiciel informatique afin de permettre une analyse de présence des micro-organismes au moyen d'un ordinateur.

Le moyen de repérage 10 comprend un cercle C1 dont les dimensions sont du même ordre de grandeur que les dimensions d'un cercle représentant le fond d'une boîte de Pétri. Le cercle C1 comprend un centre O et un rayon RC1.

Le moyen de repérage 10 comprend également un cercle C2 concentrique au cercle C1 et de rayon RC2 tel que le rayon RC2 est inférieur au rayon RC1.

Le cercle C1 comprend également une ligne d'ensemencement LR de longueur R s'étendant dans la direction du rayon RC1 et telle que la longueur R est inférieure ou égale à la valeur de la différence entre les rayons RC1 et RC2, c'est à dire R < RC1 - RC2. La ligne d'ensemencement LR représente une ligne de référence le long de laquelle une bande de fluide à analyser est inoculée sur le milieu de culture.

Un dispositif d'ensemencement permet le déplacement du fluide depuis la ligne d'ensemencement LR, sur la totalité ou une partie de la surface du milieu de culture gélosé du fond de la boîte de Pétri. La longueur R de la ligne d'ensemencement LR est généralement inférieure ou égale à la largeur du dispositif d'ensemencement. Le dispositif d'ensemencement comprend une zone de contact avec le milieu de culture gélosé. La zone de contact comprend au moins un point de contact avec le milieu de culture gélosé par l'intermédiaire du fluide localisé entre le point de contact et le milieu de culture. La zone de contact peut être, par exemple, continue ou discontinue.

Le dispositif d'ensemencement comprend par exemple un peigne tel que décrit dans les demandes de brevet WO2005/071055 et WO2008/093439. Chaque dent du peigne correspond à un point de contact. Ainsi, l'ensemble des dents du peigne représente une zone de contact discontinue avec le milieu de culture.

Le dispositif d'ensemencement peut également comprendre une spatule à extrémité recourbée telle que décrite dans le modèle US D582,564. Ainsi, l'extrémité recourbée représente une zone de contact continue du dispositif d'ensemencement avec le milieu de culture gélosé.

Lors de la répartition du fluide sur le milieu de culture au moyen du dispositif d'ensemencement, chaque point de contact de la zone de contact du dispositif d'ensemencement parcourt une distance azimutale à partir de la ligne d'ensemencement LR. Cette distance azimutale varie selon le point de contact considéré de la zone de contact du dispositif d'ensemencement.

Le moyen de repérage 10 comprend également un ensemble de zones de repérage Zn qui couvrent la totalité ou une partie de la surface du milieu de culture gélosée comprise dans la surface délimitée par les cercles de rayons RC1 et RC2. Ces zones sont numérotées par exemple de Z1 à Z7. Chaque zone de repérage Zn comprend une ligne de limite de zone LZ permettant de séparer deux zones de repérage adjacentes. Le disque associé au cercle C2 représente une surface non utilisable pour le dispositif d'ensemencement. En effet, pour des valeurs de rayon inférieures à la valeur de RC2, dans le disque de rayon RC2, les distances azimutales parcourues par les points de contact de la zone de contact du dispositif d'ensemencement sont trop faibles pour permettre de déterminer la concentration en micro-organismes en utilisant le moyen de repérage selon la présente invention.

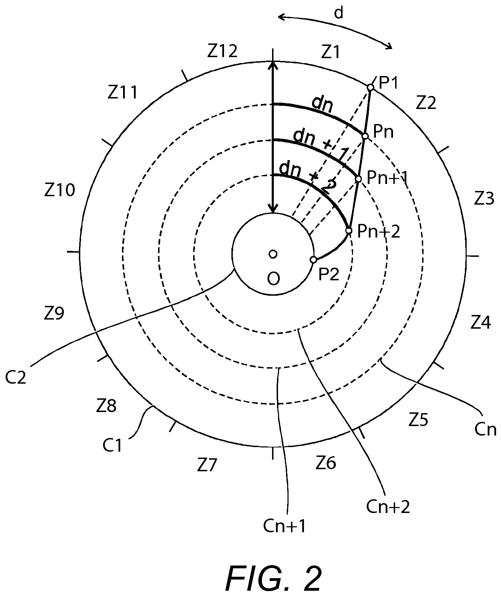

Les lignes de limite de zone LZ sont référencées par exemple de LZ1 à LZ7. Ces lignes sont réalisées sur le moyen de repérage 10 en utilisant un procédé illustré sur la figure 2.

La figure 2 montre le moyen de repérage 10 lors de son élaboration pour obtenir la représentation des lignes de limite de zone LZ.

Le moyen de repérage 10 comprend les cercles C1 et C2 comme précédemment indiqué.

Afin d'élaborer le moyen de repérage 10, des cercles Cn concentriques de rayon RCn et de centre O sont tracés sur le moyen de repérage 10. Les cercles Cn sont tels que chaque rayon RCn est inférieur au rayon RC1 et supérieur au rayon RC2. La présence de la représentation graphique des cercles Cn n'est pas nécessaire lors de l'utilisation ultérieure du moyen de repérage 10.

La circonférence du cercle C1 est divisée en un nombre déterminé de portions de cercle où chaque portion de cercle représente une distance azimutale d. La distance azimutale d correspond à un déplacement des points de contact de la zone de contact du dispositif d'ensemencement depuis la ligne d'ensemencement LR, lors d'une rotation du dispositif d'ensemencement. On définit Ai comme étant l'angle réel ou effectif de rotation du dispositif d'ensemencement sur le cercle C1 par rapport à la ligne d'ensemencement LR. Chaque distance azimutale d sur le cercle C1 délimite les zones de repérage telles que Z1 à Z7. Ainsi, chaque zone de repérage Zn est délimitée, sur la circonférence du cercle C1, par une distance azimutale qui est un multiple de la distance azimutale d et de préférence égale à la distance azimutale d.

Ainsi, de manière connue, la portion d représente une distance azimutale égale au produit de la distance R, inférieure ou égale à la différence RC1 - RC2 et représentant la longueur de la ligne d'ensemencement LR, par l'angle de rotation Ai prédéterminé et associé à la rotation du dispositif d'ensemencement lors du déplacement du dispositif d'ensemencement sur la circonférence du cercle C1. Ainsi, on obtient d = R x Ai.

Un des buts de la présente invention est de fournir un moyen de repérage 10 permettant de repérer les zones du milieu de culture dans lesquelles des colonies isolées de micro-organismes sont observées. Ainsi, le moyen de repérage 10 permet de déterminer la concentration initiale de micro-organismes dans le fluide d'intérêt, c'est-à-dire le liquide porteur comprenant les bactéries.

Pour chaque cercle Cn, une distance azimutale dn est repérée. Cette distance dn est la représentation de la distance azimutale parcourue par les points de contact de la zone de contact du dispositif d'ensemencement sur un cercle Cn spécifique lors de la rotation du dispositif d'ensemencement d'un angle Ai.

La distance azimutale dn est donc égale à un multiple de la distance azimutale d, dont la valeur est définie par une valeur déterminée. Ainsi, en se référant à la valeur connue de d, on obtient dn = (R x Ai) x k où k est un nombre représentant le coefficient relatif à la multiplicité de d par rapport à dn.

On définit Aj comme étant l'angle fictif de rotation du dispositif d'ensemencement sur un cercle Cn par rapport à la ligne d'ensemencement LR pour un point de contact situé à une distance Rn du centre O, tel que la distance azimutale dn soit égale à d pour un cercle Cn spécifique.

La distance azimutale dn est aussi égale au produit du rayon Rn par l'angle de rotation Aj associé à la rotation d'un point de contact de la zone de contact du dispositif d'ensemencement, soit dn = Rn x Aj. La valeur de dn correspond à la distance parcourue par un point de contact de la zone de contact du dispositif d'ensemencement sur le cercle concentrique Cn lors de la rotation du dispositif d'ensemencement selon l'angle Aj. Chaque portion dn permet de représenter la distance ou le chemin que chaque point de contact de la zone de contact doit parcourir sur un cercle Cn donné pour obtenir une distance azimutale parcourue et relative à la zone Zn considérée telle que dn est égale à d.

La valeur de d est donc déterminable dans la mesure où les valeurs de l'angle de rotation Ai du dispositif d'ensemencement et du rayon R associé à la longueur de la ligne d'ensemencement sont connues. En utilisant Aj = dn/Rn, on obtient la valeur de l'angle de rotation Aj. Ainsi, sur le cercle Cn, pour une rotation d'angle Aj à partir de la ligne d'ensemencement LR, on obtient un point Pn qui représente l'extrémité de la portion dn sur la circonférence correspondante du cercle Cn.

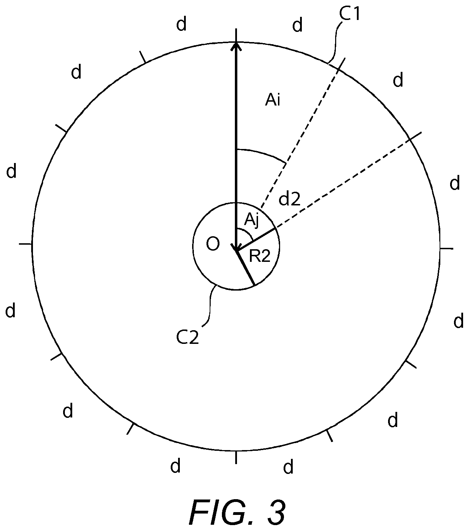

Ainsi, comme montré sur la figure 3, une rotation du dispositif d'ensemencement d'un angle Ai représenté par une portion d sur le cercle C1 correspond à une rotation du peigne d'un angle Aj pour obtenir une portion de longueur d2 identique à d sur le cercle C2.

On obtient donc Aj = d2/RC2, avec d2 = d = Ai x R, ce qui permet de définir l'angle Aj associé à la distance d2 sur le cercle C2, tel que Aj = (Ai x R) / RC2.

Ainsi, en répétant ce procédé sur l'ensemble des cercles Cn, on obtient un point Pn+1 sur le cercle Cn+1, un point Pn+2 sur le cercle Cn+2, et ainsi de suite sur l'ensemble des cercles Cn comme montré sur la figure 2.

Pour chaque rotation du dispositif d'ensemencement on définit les points P1 et P2 associés sur les cercles C1 et C2.

Pour une même rotation d'angle Ai l'ensemble des points Pn, Pn+1, Pn+2, etc. associés à une même zone Zn, sont reliés ensemble comme montré sur la figure 2 pour former une ligne. L'extrémité de cette ligne est reliée aux points P1 et P2, respectivement localisés sur les cercles C1 et C2, pour obtenir la représentation d'une zone de repérage Zn comme montré sur la figure 2. La zone de repérage Zn contient une quantité de fluide associée à un déplacement du dispositif d'ensemencement sur une même distance d.

Ainsi, lors de la rotation d'un angle Ai du dispositif d'ensemencement, le point de contact de la zone de contact parcourt une distance azimutale d sur la circonférence du cercle C1. La zone Z1 permet de repérer la quantité de fluide ayant parcouru la même distance d depuis la ligne d'ensemencement LR. Chaque zone Zn est repérée sur la circonférence du cercle C1 par une distance azimutale égale à d.

Le dispositif d'ensemencement effectue un nombre déterminé de rotations d'angle Ai vis-à-vis du fond de la boîte de Pétri. Ces rotations d'angle Ai permettent d'étaler sur le milieu de culture la totalité du fluide contenu dans la ligne d'ensemencement LR.

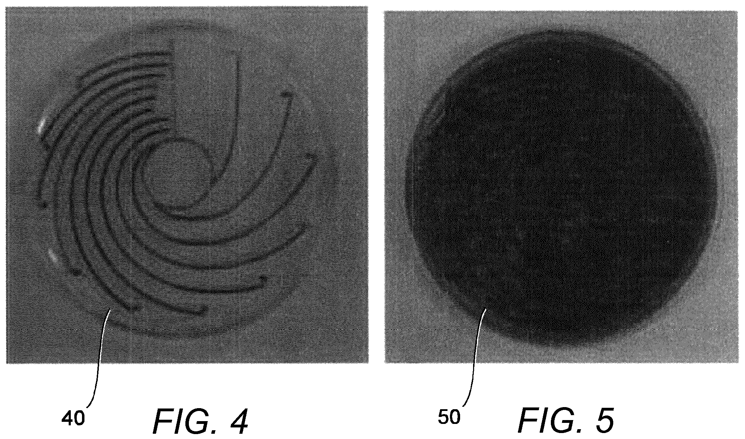

De manière pratique, le moyen de repérage 10 peut faire l'objet d'un support indépendant à appliquer sur le couvercle d'une boîte de Pétri après incubation de la boîte de Pétri. Alternativement, un couvercle d'une boîte de Pétri peut intégrer une représentation graphique du moyen de repérage 10. Ainsi, la figure 4 montre un couvercle 40 d'une boîte de Pétri intégrant une représentation graphique des différentes zones de repérage. La figure 5 montre un fond 50 d'une boîte de Pétri comprenant un milieu de culture gélosé. Ainsi, le couvercle 40 peut être disposé sur le fond 50 afin de procéder au repérage des micro-organismes sur le milieu de culture gélosé.

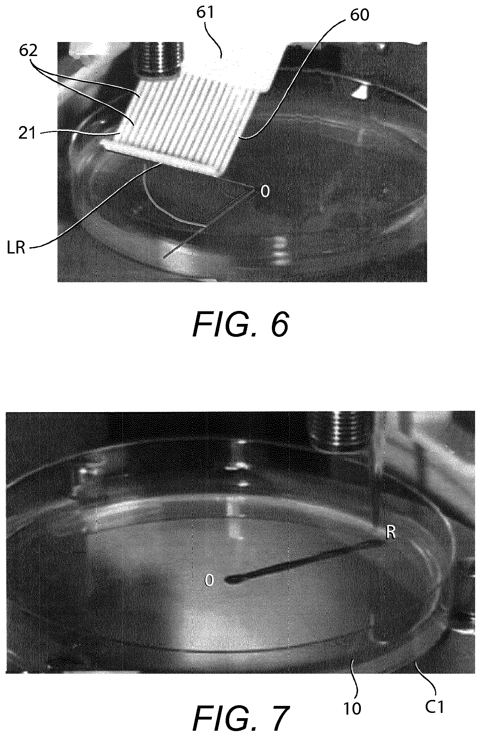

Comme montré sur la figure 6, un dispositif d'ensemencement tel qu'un peigne 60 est disposé au-dessus du fond 50 d'une boîte de Pétri. Le fond 50 est représenté par un cercle C1 de centre O. Le fond 50 peut être posé sur un support capable de se déplacer selon un mouvement circulaire afin de produire une rotation du fond 50. Le fond 50 contient un milieu de culture tel qu'un milieu de culture gélosé. Une ligne d'ensemencement LR de longueur R est disposée sur le milieu de culture.

Le peigne 60 comprend un support de dents 61 pourvus d'une pluralité de dents 62 comprenant au moins une dent. De manière préférentielle, le peigne 60 comprend 17 dents. Chaque dent 62 est séparée d'une dent adjacente par une même distance. La largeur totale du peigne 60 correspond à une valeur inférieure à la longueur R de la ligne d'ensemencement. Le peigne 60 est mobile en rotation par rapport au fond 50 de la boîte de Pétri.

Le procédé de repérage selon l'invention permet de repérer des micro-organismes présents au sein d'un fluide sur le milieu de culture, après incubation de la boîte de Pétri. Le fluide comprend, par exemple, un échantillon biologique comme précédemment mentionné. Aux fins du présent exemple, considérons l'hypothèse où la longueur R de la ligne d'ensemencement LR est égale au rayon RC1 de la boîte de Pétri.

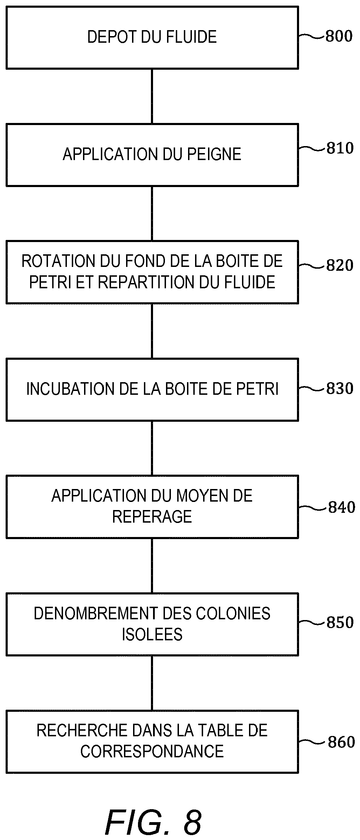

Le procédé selon l'invention comprend les étapes décrites en figure 8. Dans une étape 800, un dispositif d'inoculation (non montré), contrôlé par exemple par un programme d'ordinateur au moyen d'un ordinateur, dépose une quantité de fluide sous la forme d'une ligne d'ensemencement LR sur le milieu de culture comme indiqué en figure 7. La ligne d'ensemencement LR s'étend sur le fond 50 de la boîte de Pétri, dans la direction du rayon RC1 de la boîte de Pétri.

De manière connue, dans une étape 810, le peigne 60 est appliqué sur le fluide localisé sur la ligne d'ensemencement LR. Le peigne 60 et le fond 50 de la boîte de Pétri sont prévus pour fonctionner ensemble de la manière suivante : lors d'une rotation du fond 50, le peigne 60 entre en contact avec la ligne d'ensemencement LR. Ainsi, dans une étape 820, le fond de la boîte de Pétri entre en rotation et le peigne 60 répartit, sur le milieu de culture, le fluide contenu dans la ligne d'ensemencement LR. Le peigne 60 peut être préalablement paramétré afin d'effectuer, par rapport au fond 50 de la boîte de Pétri, des rotations répétées centrées sur le centre O du fond 50 de la boîte de Pétri et d'un même angle Ai.

Lors du déplacement du peigne, seule la dent 21 localisée sur la périphérie du fond 50 de la boîte de Pétri se déplace d'une distance azimutale d sur la circonférence du cercle C1 telle que d = R x Ai. Les autres dents 62 du peigne 60 parcourent une distance azimutale dont la longueur est inférieure à d. Ainsi, pour un même peigne 60, l'angle fictif de rotation Aj de chaque dent 62 située strictement à l'intérieur du cercle C1, doit être supérieur à l'angle réel de rotation Ai, afin que chaque dent 62 parcourt une distance azimutale identique à d sur le cercle concentrique Cn correspondant. En effet, au fur et à mesure que l'on considère une dent 62 proche du centre O du cercle C1, la distance azimutale parcourue par la dent 62 diminue, pour un angle de rotation Ai donné.

Le déplacement du peigne 60 associé au déplacement du fond de la boîte de Pétri se répète jusqu'à ce que la totalité du contenu du fluide de la ligne d'ensemencement LR soit réparti sur le milieu de culture gélosé.

Ensuite, dans une étape 830, le fond 50 de la boîte de Pétri recouvert par un couvercle adapté pour une boîte de Pétri est incubé dans un incubateur pour une période prédéterminée. Le but de l'incubation est de permettre la croissance de micro-organismes potentiellement contenus dans le fluide réparti sur le milieu de culture. La période d'incubation peut s'étendre sur quelques heures ou sur quelques jours, préférentiellement sur vingt-quatre heures. Cette période d'incubation peut être continue c'est-à-dire sans analyse intermédiaire du contenu de la boîte de Pétri, entre l'inoculation de l'échantillon biologique et la fin de la période d'incubation. La période d'incubation peut également être discontinue, c'est-à-dire avec une analyse régulière du contenu de la boîte de Pétri à des instants prédéterminés pendant la période d'incubation.

Les étapes 810, 820 et 830 précédemment décrites peuvent être réalisées au moyen d'un dispositif automatisé contrôlé par un programme d'ordinateur au moyen d'un ordinateur.

A un instant prédéterminé, soit au cours de la période d'incubation, soit à la fin de la période d'incubation, dans une étape 840, le moyen de repérage 10 selon la présente invention est appliqué sur la boîte de Pétri. L'étape 840 peut être réalisée soit de manière manuelle, soit de manière automatisée au moyen d'un dispositif mécanique contrôlé par un programme d'ordinateur, soit de manière électronique en utilisant l'image d'un moyen de repérage à appliquer sur une image de la boîte de Pétri au moyen d'un dispositif électronique tel qu'un ordinateur.

Après incubation, le milieu de culture, dans le fond 50 de la boîte de Pétri, comporte des colonies de micro-organismes localisées à différents endroits de la surface du milieu de culture. Certaines colonies peuvent apparaître isolées et donc être facilement dénombrées visuellement par l'utilisateur. D'autres colonies peuvent être amassées sans possibilité d'être dénombrées. Dans une situation où la valeur de la concentration en micro-organismes par millilitre (ml) est supérieure à une concentration de l'ordre de 10³ micro-organismes par ml, de préférence supérieure à une concentration égale à 10³ micro-organismes par ml, la distribution des colonies est représentée par des zones continues de colonies sur le milieu de culture. L'utilisation du moyen de repérage 10 selon la présente invention est alors optimale.

Dans une étape 850, l'utilisateur peut dénombrer visuellement les colonies isolées au sein des zones de repérage du moyen de repérage 10. L'étape 850 peut également être réalisée au moyen d'un dispositif automatisé contrôlé par un programme d'ordinateur relatif à un procédé de traitement d'images, en utilisant l'image d'un moyen de repérage appliquée sur une image de la boîte de Pétri, au moyen d'un dispositif électronique tel qu'un ordinateur.

Ensuite dans une étape 860, l'utilisateur recherche la valeur de la concentration associée au nombre de colonies isolées dénombrées dans une zone considérée en utilisant, par exemple, la table de correspondance ci-dessous :

| Concentration en CFU/ml (CFU : Unité Formant Colonie (Colony Forming Unit)) | Nombre de colonies isolées repérées dans une zone de repérage |
|---|---|
| 10² | Inférieur à 10 colonies |
| 10³ | Inférieur à 30 colonies |
| 10⁴ | Supérieur à 30 colonies et la zone de repérage Z1 n'est pas pleine |
| 10⁵ | Zone de repérage Z1 remplie (aucune colonie isolée) Zones de repérage Z2 et Z3 comprenant des colonies isolées |
| 10⁶ | Zone de repérage Z1 et Z2 remplies (aucune colonie isolée) Zones de repérage Z3 et Z4 comprenant des colonies isolées |
| 10⁷ | Zone de repérage Z1, Z2 et Z3 remplies (aucune colonie isolée) Zone de repérage Z4 comprenant des colonies isolées |
| 10⁸ | Zone de repérage Z1, Z2, Z3 et Z4 remplies (aucune colonie isolée) Zone de repérage Z5 comprenant des colonies isolées |

Les valeurs de concentration présentées dans le tableau ci-dessus sont obtenues en utilisant la formule mathématique C (RCn, A) = Coexp(-RCn x A/K) qui définit la distribution de concentration des micro-organismes sur le milieu de culture. Dans cette formule mathématique :
A est la valeur de l'angle réel de rotation du dispositif d'ensemencement (60) ;
Co est la concentration initiale en micro-organismes du fluide inoculé;
RCn est le rayon du cercle concentrique considéré;
K est une constante d'épuisement spécifique d'un fluide par rapport au milieu de culture gélosé sur lequel le fluide est réparti.

L'étape 860 peut être réalisée au moyen d'un ordinateur comprenant un programme d'ordinateur adapté.

Ainsi, les étapes 800, 810, 820, 830, 840, 850 et 860 peuvent être réalisées de manière automatisée.

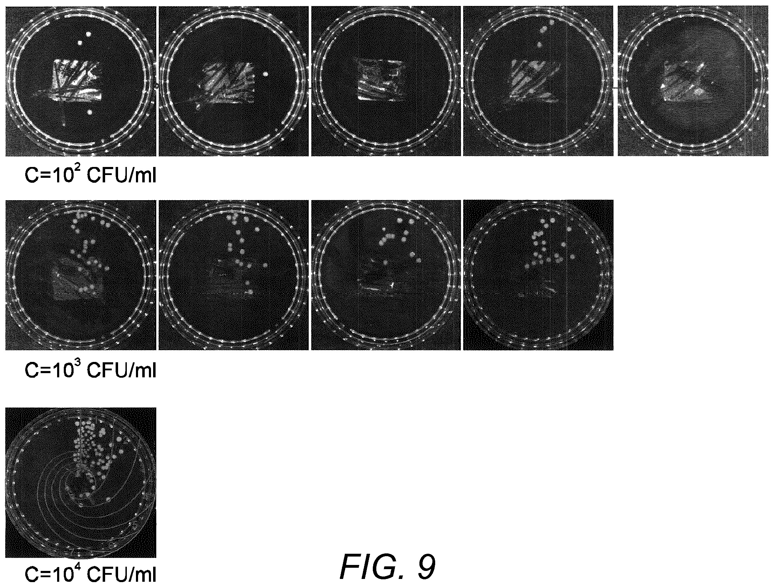

Les figures 9, 10 et 11 montrent des exemples d'application du procédé de repérage pour des échantillons urinaires.

La figure 9 montre des concentrations progressives de 10² à 10⁴ CFU/ml sur gélose TSA (Tryptic Soy Agar).

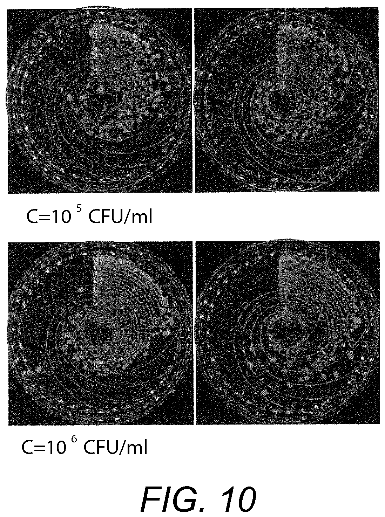

La figure 10 montre des concentrations progressives de 10⁵ à 10⁶ CFU/ml sur gélose TSA.

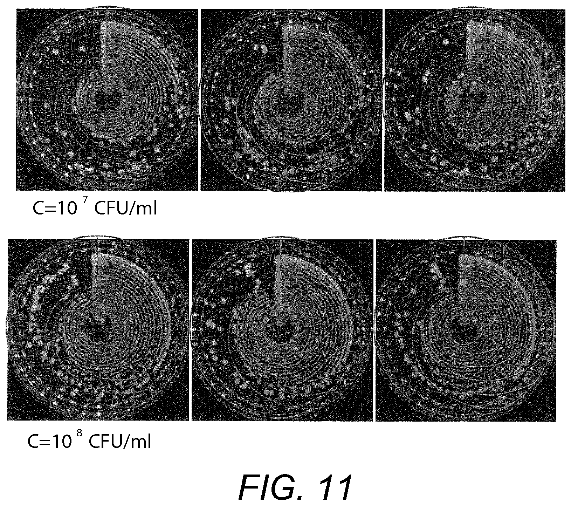

La figure 11 montre des concentrations progressives de 10⁷ à 10⁸ CFU/ml sur gélose TSA.

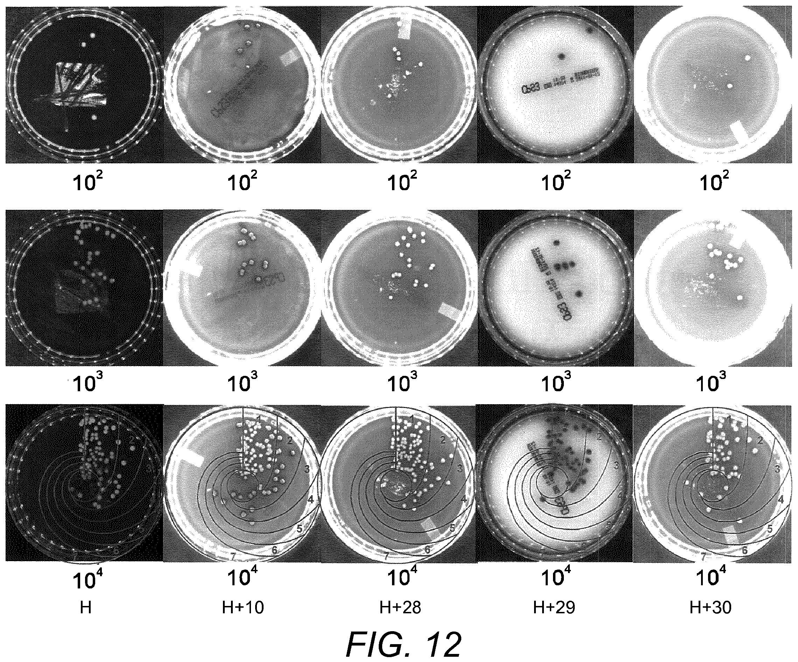

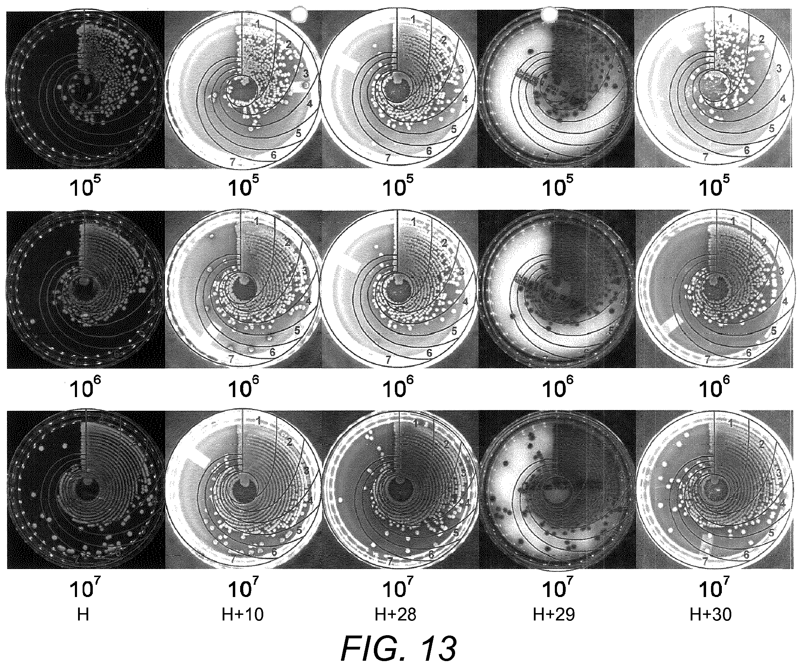

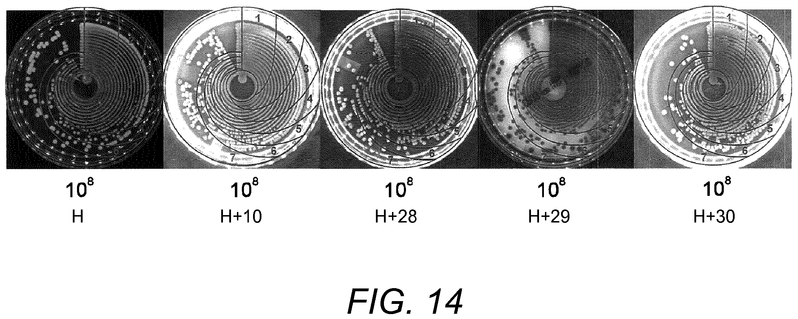

De manière similaire, les figures 12, 13 et 14 montrent un exemple d'analyse d'un même fluide à différentes concentrations (10² à 10⁸ CFU/ml) inoculé sur milieu ChromID™ CPS^{®} à différents moments successifs dans le temps. Ainsi, en se basant sur un nombre d'heures, on peut observer l'évolution de la concentration en micro-organismes à une heure H donnée puis successivement à une heure H+10 heures, H+26 heures, et ainsi de suite par exemple. Il s'agit d'une préparation de solution bactérienne extemporanée. Ainsi, la reproductibilité de la méthode de repérage peut être estimée.

Au total, 122 boîtes ont été testées avec la méthode selon la présente invention sur milieu ChromID™ CPS^{®}, c'est-à-dire un milieu dédié à la numération urinaire, pour lequel l'intérêt de cette méthode est majeur. La valeur seuil de concentration était de 10⁴ CFU/ml.

Comme montré sur le tableau ci-dessous, on obtient une sensibilité et une spécificité de 100% par rapport à la concentration bactérienne théorique.

| | | Estimation avec le moyen de repérage selon la présente invention | | | |
|---|---|---|---|---|---|
| | | Négatif | Incertitude | Positif | Total |
| T h é o r i e | Négatif (<10⁴ CFU/ml) | 40 | 0 | 0 | 40 |
| | Incertitude (=10⁴ CFU/ml) | 0 | 18 | 0 | 18 |
| | Positif (>10⁴ CFU/ml) | 0 | 0 | 59 | 59 |
| | Total | 40 | 18 | 59 | 117 |

La présente invention est spécifiquement adaptée aux dispositifs d'ensemencement précédemment décrits ou à des dispositifs d'ensemencement similaires dans leur mode de fonctionnement concernant la répartition du fluide sur un milieu de culture adapté au mode de répartition.

De tels dispositifs d'ensemencement comprennent, par exemple, des dispositifs mobiles en rotation par rapport au centre de la boîte de Pétri et permettant un dépôt radial du fluide au moyen d'une extension radiale.

## Revendications

1. Moyen de repérage (10) adapté pour déterminer la concentration en micro-organismes d'un fluide à analyser au moyen d'une boîte de Pétri après incubation de ladite boite de Pétri, ladite boîte de Pétri comprenant un couvercle et un fond, le moyen de repérage étant adapté pour être posé sur le couvercle de la boîte de Pétri ou intégré au couvercle de la boîte de Pétri ou intégré au sein d'un logiciel informatique, ladite boîte de Pétri comprenant un milieu de culture sur lequel un dispositif d'ensemencement (60) mobile en rotation par rapport à la boîte de Pétri est adapté pour répartir le fluide, ledit dispositif d'ensemencement (60) comprenant au moins un point de contact, associé à une zone de contact, avec ledit milieu de culture, ledit moyen de repérage (10) comprenant :
- un cercle C1 correspondant à la circonférence de la boîte de Pétri, le cercle C1 comprenant un centre O et un rayon RC1 correspondant au rayon de la boîte de Pétri ;
- une ligne d'ensemencement LR de longueur R représentant la zone d'inoculation du fluide sur le milieu de culture et correspondant à une portion du rayon RC1 de la boîte de Pétri ;
- au moins une zone de repérage Zn pour repérer la présence de micro-organismes sur le milieu de culture,
ladite zone de repérage Zn étant délimitée par la ligne d'ensemencement LR et une ligne de limite de zone LZ délimitant une distance identique d parcourue sur la surface du milieu de culture par chaque point de contact du dispositif d'ensemencement (60) à partir de la ligne d'ensemencement LR.

2. Moyen de repérage (10) selon la revendication 1, dans lequel le fluide comprend une concentration en micro-organismes supérieure à 10³ micro-organismes par millilitre (ml).

3. Moyen de repérage (10) selon la revendication 1 ou 2, dans lequel chaque zone de repérage Zn correspond à une valeur prédéterminée de la concentration en micro-organismes du fluide.

4. Moyen de repérage (10) selon l'une des revendications précédentes, dans lequel la valeur prédéterminée est définie par une fonction exponentielle de l'angle de rotation du dispositif d'ensemencement (60), de la longueur R de la ligne d'ensemencement LR, de la boîte de Pétri et d'une constante définissant la vitesse d'épuisement de la concentration bactérienne dans le fluide en fonction de la distance parcourue sur le milieu de culture.

5. Moyen de repérage (10) selon l'une des revendications précédentes, dans lequel la zone de repérage Zn est délimitée par le cercle C1 et par un cercle C2 de rayon RC2 concentrique au cercle C1 et tel que RC2 est inférieur à RC1.

6. Moyen de repérage (10) selon la revendication 5, dans lequel la ligne de limite de zone LZ est formée par un ensemble de points Pn, chaque point Pn étant situé sur un cercle Cn concentrique au cercle C1, ledit cercle Cn comprenant un rayon RCn tel que RCn est inférieur à RC1 et supérieur à RC2, et chaque point Pn étant situé à une distance égale de la ligne d'ensemencement LR pour une même ligne de limite de zone LZ.

7. Moyen de repérage selon l'une des revendications précédentes, dans lequel les micro-organismes comprennent des bactéries.

8. Procédé de repérage pour déterminer la concentration en micro-organismes d'un fluide à analyser au moyen d'une boîte de Pétri de rayon RC1, ladite boîte de Pétri comprenant un milieu de culture avec :
a) un fluide déposé sur le milieu de culture pour former une ligne d'ensemencement LR correspondant à une portion du rayon RC1 de la boîte de Pétri au moyen d'un dispositif d'inoculation;
b) ledit le fluide contenu dans la ligne d'ensemencement LR étant réparti sur le milieu de culture au moyen d'un dispositif d'ensemencement (60) mobile en rotation par rapport à la boîte de Pétri, ledit dispositif d'ensemencement (60) comprenant au moins un point de contact avec le milieu de culture ;
c) la boîte de Pétri étant incubée dans un incubateur pour permettre la croissance des micro-organismes sur le milieu de culture, ledit procédé comprenant, après incubation de la boîte de Pétri dans l'incubateur, l'étape suivante d) :
d) identifier des zones de repérage Zn adjacentes pour repérer la présence des micro-organismes sur le milieu de culture, lesdites zones de repérage Zn couvrant au moins une partie de la surface de la boîte de Pétri,
et
une première zone de repérage Zn étant délimitée par la ligne d'ensemencement LR et une ligne de limite de zone LZ délimitant une même distance d parcourue sur le milieu de culture, par chaque point de contact du dispositif d'ensemencement (60) à partir de la ligne d'ensemencement LR.

9. Procédé de repérage selon la revendication 8, dans lequel le fluide comprend une concentration en micro-organismes supérieure à 10³ micro-organismes par millilitre (ml).

10. Procédé de repérage selon la revendication 8 ou 9, ledit procédé comprenant l'étape suivante :
e) identifier une zone de repérage Zn comprenant des colonies isolées de micro-organismes.

11. Procédé de repérage selon la revendication 10, ledit procédé comprenant l'étape suivante :
f) retrouver dans une table de correspondance une valeur prédéterminée de concentration en micro-organismes associée à la zone de repérage Zn identifiée afin de déterminer la concentration des micro-organismes dans le fluide.

12. Procédé de repérage selon l'une des revendications 8 à 11, ledit procédé mettant en oeuvre le moyen de repérage (10) selon l'une des revendications 1 à 7.

13. Procédé de repérage selon la revendication 12, dans lequel ledit moyen de repérage (10) est adapté pour être intégré au sein d'un logiciel informatique, ledit procédé étant réalisé en appliquant une image dudit moyen de repérage (10) sur une image de ladite boîte de Pétri au moyen d'un dispositif électronique tel qu'un ordinateur.

14. Produit-programme d'ordinateur comprenant des instructions logicielles pour la mise en oeuvre d'un procédé selon l'une des revendications 8 à 13 lorsque ledit programme est exécuté par un processeur de données.

## Patentansprüche

1. Ein Ortungsmittel (10), das geeignet ist, um die Konzentration von Mikroorganismen eines mittels einer Petrischale nach der Inkubation der Petrischale zu analysierenden Fluids zu bestimmen, wobei die Petrischale einen Deckel und einen Boden beinhaltet, wobei das Ortungsmittel geeignet ist, um auf den Deckel der Petrischale gesetzt oder in den Deckel der Petrischale integriert oder innerhalb eines Computerprogramms integriert zu werden, wobei die Petrischale ein Kulturmedium beinhaltet, auf dem eine Beimpfungsvorrichtung (60), die drehbar in Bezug auf die Petrischale bewegbar ist, geeignet ist, um das Fluid zu verteilen, wobei die Beimpfungsvorrichtung (60) mindestens einen mit einer Kontaktzone assoziierten Kontaktpunkt mit dem Kulturmedium beinhaltet, wobei das Ortungsmittel (10) Folgendes beinhaltet:
- einen Kreis C1, der dem Umfang der Petrischale entspricht, wobei der Kreis C1 einen Mittelpunkt O und einen Radius RC1, welcher dem Radius der Petrischale entspricht, beinhaltet;
- eine Beimpfungslinie LR mit einer Länge R, welche die Inokulationszone des Fluids auf das Kulturmedium darstellt und einem Abschnitt des Radius RC1 der Petrischale entspricht;
- mindestens eine Ortungszone Zn zum Orten des Vorhandenseins von Mikroorganismen auf dem Kulturmedium,
wobei die Ortungszone Zn durch die Beimpfungslinie LR und eine Zonenbegrenzungslinie LZ begrenzt ist, welche eine identische Entfernung d begrenzt, die über die Oberfläche des Kulturmediums durch jeden Kontaktpunkt der Beimpfungsvorrichtung (60) ab der Beimpfungslinie LR zurückgelegt wird.

2. Ortungsmittel (10) gemäß Anspruch 1, wobei das Fluid eine Konzentration von Mikroorganismen von mehr als 10³ Mikroorganismen pro Milliliter (ml) beinhaltet.

3. Ortungsmittel (10) gemäß Anspruch 1 oder 2, wobei jede Ortungszone Zn einem vorher festgelegten Wert der Konzentration von Mikroorganismen des Fluids entspricht.

4. Ortungsmittel (10) gemäß einem der vorhergehenden Ansprüche, wobei der vorher festgelegte Wert durch eine exponentielle Funktion des Drehwinkels der Beimpfungsvorrichtung (60), der Länge R der Beimpfungslinie LR, der Petrischale und einer Konstante, welche die Rate der Erschöpfung der Bakterienkonzentration in dem Fluid als eine Funktion der auf dem Kulturmedium zurückgelegten Entfernung definiert, definiert ist.

5. Ortungsmittel (10) gemäß einem der vorhergehenden Ansprüche, wobei die Ortungszone Zn durch den Kreis C1 und einen Kreis C2 mit Radius RC2, der konzentrisch mit dem Kreis C1 ist, begrenzt ist, und sodass RC2 kleiner als RC1 ist.

6. Ortungsmittel (10) gemäß Anspruch 5, wobei die Zonenbegrenzungslinie LZ durch einen Satz Punkte Pn gebildet wird, wobei sich jeder Punkt Pn auf einem mit Kreis C1 konzentrischen Kreis Cn befindet, wobei der Kreis Cn einen Radius RCn beinhaltet, sodass RCn kleiner als RC1 und größer als RC2 ist, und wobei sich jeder Punkt Pn mit einer gleichen Entfernung von der Beimpfungslinie LR für eine gleiche Zonenbegrenzungslinie LZ befindet.

7. Ortungsmittel gemäß einem der vorhergehenden Ansprüche, wobei die Mikroorganismen Bakterien beinhalten.

8. Ein Ortungsverfahren zur Bestimmung der Konzentration von Mikroorganismen eines mittels einer Petrischale mit Radius RC1 zu analysierenden Fluids, wobei die Petrischale ein Kulturmedium mit Folgendem beinhaltet:
a) einem Fluid, das auf dem Kulturmedium zum Formen einer Beimpfungslinie LR, die einem Abschnitt des Radius RC1 der Petrischale entspricht, mittels einer Inokulationsvorrichtung angeordnet wird;
b) wobei das Fluid, das in der Beimpfungslinie LR enthalten ist, auf dem Kulturmedium mittels einer Beimpfungsvorrichtung (60), die drehbar in Bezug auf die Petrischale bewegbar ist, verteilt wird, wobei die Beimpfungsvorrichtung (60) mindestens einen Kontaktpunkt mit dem Kulturmedium beinhaltet;
c) wobei die Petrischale in einem Inkubator inkubiert wird, um das Wachsen der Mikroorganismen auf dem Kulturmedium zu ermöglichen, wobei das Verfahren nach der Inkubation der Petrischale in dem Inkubator den folgenden Schritt d) beinhaltet:
d) Identifizieren benachbarter Ortungszonen Zn zum Orten der Anwesenheit der Mikroorganismen auf dem Kulturmedium, wobei die Ortungszonen Zn mindestens einen Teil der Oberfläche der Petrischale abdecken, und
wobei eine erste Ortungszone Zn durch die Beimpfungslinie LR und eine Zonenbegrenzungslinie LZ begrenzt ist, welche eine gleiche Entfernung d begrenzt, die über das Kulturmedium durch jeden Kontaktpunkt der Beimpfungsvorrichtung (60) ab der Beimpfungslinie LR zurückgelegt wird.

9. Ortungsverfahren (10) gemäß Anspruch 8, wobei das Fluid eine Konzentration von Mikroorganismen von mehr als 10³ Mikroorganismen pro Milliliter (ml) beinhaltet.

10. Ortungsverfahren gemäß Anspruch 8 oder 9, wobei das Verfahren den folgenden Schritt beinhaltet:
e) Identifizieren einer Ortungszone Zn, die von Mikroorganismen isolierte Kolonien beinhaltet.

11. Ortungsverfahren gemäß Anspruch 10, wobei das Verfahren den folgenden Schritt beinhaltet:
f) Wiederfinden in einer Zustandstabelle eines vorher festgelegten Wertes der Konzentration von Mikroorganismen, die mit der identifizierten Ortungszone Zn assoziiert sind, um die Konzentration von Mikroorganismen in dem Fluid zu bestimmen.

12. Ortungsverfahren gemäß einem der Ansprüche 8 bis 11, wobei das Verfahren das Ortungsmittel (10) gemäß einem der Ansprüche 1 bis 7 einsetzt.

13. Ortungsverfahren gemäß Anspruch 12, wobei das Ortungsmittel (10) geeignet ist, um innerhalb eines Computerprogramms integriert zu werden, wobei das Verfahren durchgeführt wird, indem ein Bild des Ortungsmittels (10) auf ein Bild der Petrischale mit Hilfe einer elektronischen Vorrichtung wie etwa einem Computer aufgebracht wird.

14. Ein Computerprogrammprodukt, das Software-Anweisungen zur Durchführung eines Verfahrens gemäß einem der Ansprüche 8 bis 13, wenn das Programm durch einen Datenprozessor ausgeführt wird, beinhaltet.

## Claims

1. A locating means (10) suitable for determining the microorganism concentration of a fluid to be analysed by means of a Petri dish, after incubation of said Petri dish, said Petri dish comprising a lid and a base, the locating means being suitable to be placed on the lid of the Petri dish or integrated in the lid of the Petri dish or integrated within a computer software, said Petri dish comprising a culture medium on which a seeding device (60) able to rotate relative to the Petri dish is suitable so as to distribute the fluid, said seeding device (60) comprising at least one point of contact, associated with a contact zone, with said culture medium, said locating means (10) comprising:
- a circle C1 corresponding to the circumference of the Petri dish, the circle C1 comprising a centre O and a radius RC1 corresponding to the radius of the Petri dish;
- a seeding line LR of length R representing the zone for inoculating the fluid on the culture medium and corresponding to a portion of the radius RC1 of the Petri dish;
- at least one locating zone Zn for locating the presence of microorganisms on the culture medium,
said locating zone Zn being delimited by the seeding line LR and a zone boundary line LZ delimiting an identical distance d travelled on the surface of the culture medium by each point of contact of the seeding device (60) starting from the seeding line LR.

2. The locating means (10) according to claim 1, wherein the fluid comprises a microorganism concentration greater than 10³ microorganisms per millilitre (ml).

3. The locating means (10) according to claim 1 or 2, wherein each locating zone Zn corresponds to a predetermined value of the microorganism concentration of the fluid.

4. The locating means (10) according to one of the preceding claims, wherein the predetermined value is defined by an exponential function of the angle of rotation of the seeding device (60), of the length R of the seeding line LR, of the Petri dish and of a constant defining the depletion rate of the bacterial concentration in the fluid as a function of the distance travelled on the culture medium.

5. The locating means (10) according to one of the preceding claims, wherein the locating zone Zn is delimited by the circle C1 and by a circle C2 of radius RC2 which is concentric to circle C1, and such that RC2 is smaller than RC1.

6. The locating means (10) according to claim 5, wherein the zone boundary line LZ is formed by a set of points Pn, each point Pn being situated on a circle Cn concentric to circle C1, said circle Cn comprising a radius RCn, such that RCn is smaller than RC1 and greater than RC2, and each point Pn being situated at an equal distance from the seeding line LR for a same zone boundary line LZ.

7. The locating means according to one of the preceding claims, wherein the microorganisms comprise bacteria.

8. A locating method for determining the microorganism concentration of a fluid to be analysed by means of a Petri dish with radius RC1, said Petri dish comprising a culture medium with:
a) a fluid deposited on the culture medium to form a seeding line LR corresponding to a portion of the radius RC1 of the Petri dish by means of an inoculation device;
b) said fluid contained in the seeding line LR being distributed on the culture medium by means of a seeding device (60) able to rotate relative to the Petri dish, said seeding device (60) comprising at least one point of contact with the culture medium;
c) the Petri dish being incubated in an incubator to permit the growth of the microorganisms on the culture medium, said method comprising, after incubation of the Petri dish in the incubator, the following step d):
d) identifying adjacent locating zones Zn to locate the presence of the microorganisms on the culture medium, said locating zones Zn covering at least part of the surface of the Petri dish,
and
a first locating zone Zn being delimited by the seeding line LR and a zone boundary line LZ delimiting a same distance d travelled on the culture medium by each point of contact of the seeding device (60) starting from the seeding line LR.

9. The locating method according to claim 8, wherein the fluid comprises a microorganism concentration greater than 10³ microorganisms per millilitre (ml).

10. The locating method according to claim 8 or 9, said method comprising the following step:
e) identifying a locating zone Zn comprising isolated colonies of microorganisms.

11. The locating method according to claim 10, said method comprising the following step:
f) finding, in a correspondence table, a predetermined value for microorganism concentration associated with the identified locating zone Zn in order to determine the microorganism concentration in the fluid.

12. The locating method according to one of claims 8 to 11, said method implementing the locating means (10) according to one of claims 1 to 7.

13. The locating method according to claim 12, wherein said locating means (10) is suitable to be integrated within a computer software, said method being carried out by applying an image of said locating means (10) onto an image of said Petri dish by means of an electronic device such as a computer.

14. A computer software package comprising software instructions for implementing a method according to one of claims 8 to 13 when said program is executed by a data processor.
